(19) Europäisches Patentamt
European Patent Office
Office européen des brevets

(11) Veröffentlichungsnummer: **0 449 009 A2**

# (12) EUROPÄISCHE PATENTANMELDUNG

(21) Anmeldenummer: **91103535.0**

(51) Int. Cl.5: **A61K 31/275**

(22) Anmeldetag: **08.03.91**

(30) Priorität: **15.03.90 DE 4008323**

(43) Veröffentlichungstag der Anmeldung:
**02.10.91 Patentblatt 91/40**

(84) Benannte Vertragsstaaten:
**AT BE CH DE DK ES FR GB GR IT LI LU NL SE**

(71) Anmelder: **KNOLL AKTIENGESELLSCHAFT**
**Knollstrasse 32**
**W-6700 Ludwigshafen(DE)**

(72) Erfinder: **Weissbach, Markus Hugo, Dr.**
**Annastrasse 17b**
**W-6100 Darmstadt(DE)**

(74) Vertreter: **Karau, Wolfgang, Dr. et al**
**BASF Aktiengesellschaft Carl-Bosch-Strasse 38**
**W-6700 Ludwigshafen(DE)**

## (54) Verwendung von Gallopamil zur Herstellung von Arzneimitteln zur Behandlung von akutem Nierenversagen.

(57) Verwendung von Gallopamil und dessen Salzen mit physiologisch verträglichen Säuren zur Herstellung von Arzneimitteln zur Behandlung von akutem Nierenversagen.

EP 0 449 009 A2

Die vorliegende Erfindung betrifft die Verwendung von Gallopamil (1-(3,4-Dimethoxyphenyl)-3-methylaza-7-cyan-7-(3,4,5-trimethoxyphenyl)-8-methylnonan)

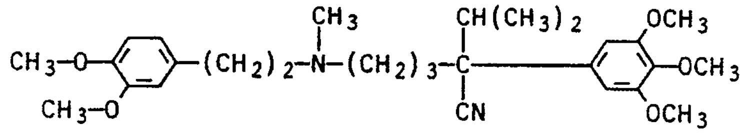

und dessen Salzen mit physiologisch verträglichen Säuren zur Herstellung von Arzneimitteln zur Behandlung von akutem Nierenversagen.

Das akute Nierenversagen ist eine selten auftretende, auf verschiedenen Ursachen beruhende schwere Erkrankung der Niere mit einer Sterblichkeitsrate von bis zu 60 %. Akutes Nierenversagen führt zur Einstellung der Urinausscheidung und zu einem Anstieg der Konzentration von harnpflichtigen Substanzen wie Kreatinin oder Harnstoff. Die Konzentration von Kreatinin kann deshalb als Indikator zur Beurteilung des Verlaufs der Krankheit verwendet werden. Akutes Nierenversagen tritt in Verbindung mit unterschiedlichen Gesundheitsstörungen wie Malaria oder verschiedenen Schockzuständen auf.

Zur Behandlung von akutem Nierenversagen kam bislang nur die zeitaufwendige Dialyse des erkrankten Patienten in Frage.

Gallopamil ist ein bekannter Wirkstoff (DE-A-1 154 810), der zur Behandlung von Hypertonie und koronarer Herzkrankheit eingesetzt wird .

Weiterhin ist aus der DE-A 1 154 810 Verapamil (1-(3,4-Dimethoxyphenyl)-3-methylaza-7-cyan-7-(3,4-dimethoxyphenyl)-8-methylnonan) bekannt, welches auf seine Wirkung auf das akute Nierenversagen im Tiermodell und beim Menschen untersucht wurde (Surg., Band 94, Seite 276-282, (1983); Kidney Int. Band 25, Seite 239, (1984); Proc. Soc. Exper. Bio. Med., Band 272, Seite 384-392 (1983); Intern. Med., Band 4, Nr. 1, Seite 15-21 (1984)).

Der Erfindung lag die Aufgabe zugrunde, ein Mittel für die Behandlung von akutem Nierenversagen bereitzustellen, welches ein günstigeres Wirkungsspektrum aufweist.

Demgemäß wurde die Verwendung von Gallopamil und dessen Salzen mit physiologisch verträglichen Säuren zur Herstellung von Arzneimitteln zur Behandlung von akutem Nierenversagen gefunden.

Weiterhin wurde gefunden, daß bei gleichzeitiger Gabe von Gallopamil und einem Schleifendiuretikum (Diuretika, deren Wirkung sich im wesentlichen auf die Henle'sche Schleife beschränkt), einem osmotisch wirksamen Diuretikum oder atrialem natriuretischem Peptid synergistische Effekte auftreten.

Gallopamil kann man in freier Form oder bevorzugt in Form eines Salzes mit einer physiologisch verträglichen Säure verabreichen. Als physiologisch verträgliche Säuren kommen z.B. Salzsäure, Schwefelsäure, Phosphorsäure, Essigsäure, Malonsäure, Bernsteinsäure, Fumarsäure, Maleinsäure, Zitronensäure, Weinsäure, Milchsäure, und Oxalsäure in Betracht.

Gallopamil und dessen Salze können in den gebräuchlichen galenischen Applikationsformen fest oder flüssig angewendet werden, z.B. in Form von Tabletten, Filmtabletten, Kapseln, Pulvern, Granulaten, Dragees, Suppositorien, Lösungen und Dosieraerosolen. Diese kann man in üblicher Weise herstellen. Es kann dabei mit den gebräuchlichen galenischen Hilfsmitteln, wie Tablettenbindern, Hilfsstoffen, Konservierungsmitteln, Tablettensprengmitteln, Disregulierungsmitteln, Weichmachern, Netzmitteln, Dispergiermitteln, Emulgatoren, Lösungsmitteln, (Retardierungsmitteln und/oder Antioxidantien (siehe z.B. H. Sucker et al: " Pharmazeutische Technologie", Thieme Verlag Stuttgart, (1978)). Als bevorzugte Zubereitungsform finden Lösungen des Gallopamils Verwendung. Die so erhältlichen Zubereitungen enthalten den Wirkstoff üblicherweise in einer Menge von 0,1 bis 99,0 Gew.-%.

Die therapeutisch notwendige Menge des Wirkstoffs kann man in üblicher Form oral oder parenteral, bevorzugt intravenös und insbesondere intrarenal verabreichen.

Die Dosierung hängt vom Alter, Zustand und Gewicht des Patienten sowie von der Applikationsart ab. In der Regel beträgt die tägliche Wirkstoffdosis zwischen 0,1 und 5,0 mg/kg Körpergewicht bei oraler Gabe und zwischen 0,01 und 5,0 mg/kg Körpergewicht bei parenteraler Gabe. Bei der intravenösen Applikation verabreicht man im Normalfall tägliche Dosen von 0,1 bis 5,0 mg/kg Körpergewicht und bei intrarenaler Applikation 0,01 bis 3,0 mg/kg Körpergewicht.

Durch die gleichzeitige Gabe von Gallopamil und verschiedenen Schleifendiuretika, osmotisch wirksamen Diuretika oder atrialem natriuretischem Peptid kann man synergistische Effekte erzielen. Vorteilhaft ist es, das Gallopamil mit einem Schleifendiuretikum wie Furosemid (4-Chloro-N-furfuryl-5-sulfamoyl-anthranilsäure) und Bumetanid (3-(Butylamino)-4-phenoxy-5-sulfamoyl-benzoesäure) zu verabreichen.

Die Diuretika und das atriale natriuretische Peptid kann man in freier Form oder in Form ihrer

physiologisch verträglichen Salze verabreichen.

Für die Galenik und Applikationsformen gelten die für Gallopamil gemachten Ausführungen mit der Ausnahme, daß die Diuretika und das atriale natriuretische Peptid bevorzugt intravenös verabreicht werden.

Die tägliche Wirkstoffdosis hängt vom Alter, Zustand und Gewicht des Patienten sowie der Applikationsform und der Art des Wirkstoffs ab. In der Regel beträgt sie zwischen 25 und 400 mol-%, bezogen auf die eingesetzte Menge Gallopamil.

Beispiele

Zum Nachweis der Wirksamkeit bei akutem Nierenversagen wurde Gallopamil 8 Patienten, die an akutem Nierenversagen erkrankt waren, über einen Zeitraum von 4 Stunden intrarenal in Dosierungen von 40 bis 160 µg/min kontinuierlich infundiert. Gleichzeitig wurde intravenös ein Diuretikum (Furosemid) in einer Konzentration von 40 µg/min über 24 Stunden verabreicht. Bei 2 weiteren Patienten wurde Gallopamil intravenös über Zeiträume von 3 und 24 Stunden in einer Menge von 40 µg/min infundiert.

In dieser Studie konnte bei 80 % der Patienten der deletäre Verlauf des akuten Nierenversagens gestoppt werden (siehe nachstehende Tabelle). Bei 90 % der Patienten konnte auf eine Dialyse verzichtet werden und alle Patienten überlebten das akute Nierenversagen.

Tabelle

| Auslösende Gesundheitsstörung | Dosis [µg/min] | Kreatinin-Ausscheidung [ml/min] vor der Behandlung | Kreatinin-Ausscheidung [ml/min] nach einem Behandlungstag | Kreatininkonzentration im Blut [mg/dl] vor der Behandlung | Kreatininkonzentration im Blut [mg/dl] nach sieben Behandlungstagen |
|---|---|---|---|---|---|
| Malaria | 40 | 5,3 | 14,3 | 10,4 | 3,1 |
| Malaria[1] | 50 | 2,2 | 3,7 | 12,7 | 5,5 |
| G-6-PD[1,2] | 83 | 0,8 | 1,2 | 14,1 | 14,6 |
| Schock | 83 | 3,4 | 13,2 | 4,7 | 1,1 |
| septischer Schock | 83 | 29,0 | 48,0 | 10,6 | 1,3 |
| hypovolenischer Schock | 160 | 2,3 | 16,1 | 9,6 | 2,5 |
| Schock[3] | 40/24 h | 10,4 | 17,9 | 5,5 | 2,9 |
| Rhabdomyolysis[3] | 40/3 h | 8,9 | 3,6 | 5,2 | 5,9 |
| Rhabdomyolysis | 80 | 4,0 | 5,1 | 10,1 | 9,3 |

1) Die Behandlung des Patienten mit Gallopamil begann 2 Wochen nach Beginn der Erkrankung und zweimaliger Dialyse des Patienten.
2) G-6-PD steht für Glukose-6-phosphatdehydrogenasemangel
3) Die Infusion des Gallopamils erfolgte intravenös

## Patentansprüche

1. Arzneimittel zur Behandlung von akutem Nierenversagen, enthaltend neben üblichen Träger- und Zusatzstoffen eine therapeutisch wirksame Menge von Gallopamil.

2. Arzneimittel zur Behandlung von akutem Nierenversagen gemäß Anspruch 1, enthaltend zusätzlich Schleifendiuretika, osmotisch wirksame Diuretika oder atriales natriuretisches Peptid.

3. Verwendung von Gallopamil und dessen Salzen mit physiologisch verträglichen Säuren zur Herstellung von Arzneimitteln zur Behandlung von akutem Nierenversagen.

4. Verwendung von Gallopamil und dessen Salzen mit physiologisch verträglichen Säuren in Verbindung mit Schleifendiuretika, osmotisch wirksamen Diuretika oder atrialem natriuretischem Peptid zur Herstellung von Arzneimitteln zur Behandlung von akutem Nierenversagen.